(19) Europäisches Patentamt
European Patent Office
Office européen des brevets

(11) Veröffentlichungsnummer : **0 328 980 B1**

(12) # EUROPÄISCHE PATENTSCHRIFT

(45) Veröffentlichungstag der Patentschrift :
**16.09.92 Patentblatt 92/38**

(51) Int. Cl.$^5$ : **C07C 303/26,** C07C 309/12

(21) Anmeldenummer : **89102001.8**

(22) Anmeldetag : **06.02.89**

(54) **Verfahren zur Herstellung niedrigviskoser Estersulfonatpasten.**

(30) Priorität : **13.02.88 DE 3804609**

(43) Veröffentlichungstag der Anmeldung :
**23.08.89 Patentblatt 89/34**

(45) Bekanntmachung des Hinweises auf die Patenterteilung :
**16.09.92 Patentblatt 92/38**

(84) Benannte Vertragsstaaten :
**AT BE CH DE ES FR GB GR IT LI NL SE**

(56) Entgegenhaltungen :
**EP-A- 0 186 896**
**EP-A- 0 222 237**
**DE-A- 3 319 591**
**DE-B- 1 418 887**

(73) Patentinhaber : **Henkel Kommanditgesellschaft auf Aktien**
**Postfach 1100 Henkelstrasse 67**
**W-4000 Düsseldorf 1 (DE)**

(72) Erfinder : **Fabry, Bernd, Dr.**
**Danziger Strasse 31**
**W-4052 Korschenbroich (DE)**
Erfinder : **Piorr, Robert, Dr.**
**Kieselei 12**
**W-4030 Ratingen-Hösel (DE)**

**EP 0 328 980 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung niedrigviskoser, hochkonzentrierter wäßriger Pasten von Alkalimetallsalzen α-sulfonierter Fettsäurealkylester.

Alkalimetallsalze von α-Sulfofettsäurealkylestern erhält man bekanntlich in Form wäßriger Pasten durch Neutralisation von α-Sulfofettsäureestern mit wäßrigem Alkalimetallhydroxid. Als technisches Ausgangsmaterial werden zunehmend nachwachsende Rohstoffe natürlichen Ursprungs, überwiegend Fette und/oder Öle pflanzlichen Ursprungs, verwendet, die durch Esterspaltung und nachfolgende Veresterung mit niederen Alkanolen, insbesondere Methanol, bzw. durch Umesterung der natürlichen Triglyceride mit niederen Alkanolen erhalten werden. Die anfallenden Fettsäureestergemische enthalten - je nach Ursprung des natürlichen Rohstoffs - Ester von Fettsäuren eines vergleichsweise breiten C-Zahl-Bereichs, der üblicherweise zwischen l0 und 24 für den Fettsäurealkylrest liegt. Derartige Fettsäureestergemische werden bevorzugt mit gasförmigem Schwefeltrioxid sulfiert. Dies führt zu mehr oder weniger stark verfärbten sauren Rohsulfonaten, die gebleicht und durch Neutralisation auf den pH-Bereich von etwa 6 bis 7 in Estersulfonatpasten überführt werden. In dieser Form haben sie große Bedeutung als oberflächenaktive Mittel bzw. Netzmittel für Wasch- und Reinigungszwecke.

Eine besondere Schwierigkeit in der technischen Handhabung solcher Pasten von Alkalimetallsalzen α-sulfonierter Fettsäurealkylester - im Folgenden als "Estersulfonatsalze" bezeichnetliegt in ihrem Konzentrations-/Viskositätsverhalten. Technisch anfallende Estersulfonatsalze bilden in wäßriger Abmischung nur bei vergleichsweise niedrigen Feststoffkonzentrationen, beispielsweise bis zu Feststoffgehalten von etwa 35 Gew.-%, hinreichend bewegliche, beispielsweise pumpbare, Lösungen bzw. Suspensionen, deren niedrige Viskosität einen störungsfreien Ablauf technischer Vorgänge noch gewährleistet. Bei höheren Estersulfonatsalzgehalten, beispielsweise Feststoffgehalten von etwa 40 Gew.-% oder mehr, steigt die Viskosität der wäßrigen Zubereitung so stark an, daß ihre freie Beweglichkeit nicht mehr besteht. Hieraus resultieren schwerwiegende Einschränkungen: Das Ziel, unmittelbar durch Neutralisation des Rohsulfonsäuregemisches mit konzentrierter Alkalimetallhydroxidlösung hochkonzentrierte Estersulfonatsalzpasten zu gewinnen, kann nicht erreicht werden, weil die Rührbarkeit und damit die gleichmäßige Vermischung der beiden Komponenten des Reaktionsgemisches (Rohsulfonsäuregemisch und Alkalimetallhydroxidlösung) nicht mehr gewährleistet ist. Gleichzeitig damit wird der Abtransport der Neutralisationswärme unmöglich. Durch lokale Konzentrations- und Temperaturspitzen treten unerwünschte Nebenreaktionen ein. Nachteilig ist außerdem, daß durch den Viskositätsanstieg immobilisierte Estersulfonatpasten im großtechnischen Betrieb nicht mehr pumpbar sind. Es tritt der Verschluß von Rohrleitungen und damit eine nachhaltige Störung des Betriebs der Gesamtanlage ein.

Der Stand der Technik zu Estersulfonatsalzpasten beschäftigt sich intensiv mit derartigen Besonderheiten. Zur Verbesserung des Fließverhaltens und damit der technischen Handhabbarkeit derartiger Pasten wurde insbesondere vorgeschlagen, in technischem Maßstab erhaltenen wäßrigen Konzentraten von α-Sulfonfettsäurealkylestersalzen Fließhilfsmittel bzw. Viskositätsregler zuzusetzen. So schildert beispielsweise die DE-OS 33 05 430 die Mitverwendung von $C_8$- bis $C_{40}$-Alkoholen, die zusätzlich eine oder mehrere Hydroxylgruppen als Substituenten aufweisen können und an die bis zu 20 Mol Ethylenoxid und/oder Propylenoxid pro Mol Alkohol angelagert sein können. Derartige Viskositätsregler werden den Estersulfonatpasten in Mengen von l bis l5 Gew.-%, bezogen auf die Tensidmenge, zugefügt, wobei sich eine Viskosität des Tensidkonzentrates von höchstens l0 000 mPas bei 70 °C einstellt. Bei einer Viskosität in diesem Bereich ist die Pumpbarkeit der Mischung auch im industriellen Maßstab gewährleistet.

Die DE-OS 34 47 859 beschreibt die Verwendung von Alkalimetallalkansulfonaten mit einer mittleren Kohlenstoffzahl von ll bis 2l als Viskositätsregler für wäßrige, hochkonzentrierte technische α-Sulfofettsäureester-Alkalimetallsalz-Konzentrate, die wenigstens 30 Gew.-% des aktiven Tensids enthalten. Die Viskositätsregler werden in Mengen von 0,5 bis l0 Gew.-%, bezogen auf den Tensidgehalt, eingesetzt und stellen eine Viskosität bei 40 °C von höchstens l0 000 mPas und damit ebenfalls eine Pumpbarkeit der Tensidkonzentrate im technischen Bereich sicher.

Gemäß der DE-OS 35 38 9l0 lassen sich bei erhöhten Temperaturen bewegliche wäßrige Pasten waschaktiver α-Sulfofettsäureestersalze aus Rohsulfonsäuren dadurch herstellen, daß man die Rohsulfonsäuren vor der Einleitung in die wäßrige Alkalimetallhydroxidphase einer Nachreaktion mit einwertigen Alkoholen und/oder deren Alkoxylierungsprodukten unterwirft. Bevorzugt werden dazu monofunktionelle Alkanole mit bis zu 30 C-Atomen im Alkoholrest bzw. deren Alkoxylierungsprodukte mit bis zu 20 Alkoxyresten im Molekül eingesetzt. Auch mit Hilfe dieses Verfahrens lassen sich gerade im kritischen Konzentrationsbereich von etwa 35 bis 60 Gew.-% Feststoff leicht zu bewegende Estersulfonatsalzpasten erhalten, die auch im industriellen Maßstab leicht zu verarbeiten sind.

Die Nachteile der vom Stand der Technik vorgeschlagenen Lösungen des Problems, die Viskosität von Estersulfonatsalzpasten durch Zusatz von Alkoholen zu erniedrigen, sind darin zu sehen, daß die neutralisierten Verfahrensprodukte den zur Einstellung niedriger Viskositätswerte zugesetzten Alkohol in hohen Mengen enthalten. Das ist deswegen unerwünscht, weil der kurzkettige Alkohol die Herstellung von Waschmittelgemischen durch Sprühtrocknung stört. Insbesondere wird durch die Gegenwart der Alkoholzusätze das unerwünschte "Pluming" ausgelöst. Die freien Alkohole im Tensidgemisch weisen darüberhinaus auch einen unerwünschten Fremdgeruch auf, so daß eine Desodorierung des Produktes erforderlich ist. Gegebenenfalls müssen vor einem Versprühen der wäßrigen Pasten für die Waschmittelherstellung deswegen Verfahrensschritte zur Vertreibung überschüssigen Alkohols zwischengeschalten werden. Dies verteuert jedoch das Gesamtverfahren in unerwünschter Weise.

Es war daher Aufgabe der vorliegenden Erfindung, ein Verfahren zur Herstellung von Estersulfonatsalzpasten zur Verfügung zu stellen, in dem eine Verbesserung des Fließverhaltens der Pasten bei hohen Aktivsubstanzkonzentrationen erreicht werden kann. Dabei sollten Verbindungen oder Verbindungsgemische zur Einstellung der Viskosität verwendet werden, die schon in kleinen Konzentrationen eine signifikante Viskositätsminderung bewirken. Diese Substanzen sollten jedoch die Produkteigenschaften nicht oder zumindest nicht nachteilig beeinflussen.

Die Lösung der Aufgabe besteht darin, im Herstellungverfahren als Substanzen zur Verminderung der Viskosität sogenannte Hydroxyocenolsulfonate und/oder Hydroxyocenolethersulfonate in niedrigen Konzentrationen einzusetzen und damit eine deutliche Viskositätserniedrigung von wäßrigen Estersulfonsatsalzpasten hohen Aktivsubstanzgehalts herbeizuführen.

Die Erfindung betrifft ein Verfahren zur Herstellung niedrigviskoser, hochkonzentrierter wäßriger Pasten von Alkalimetallsalzen α-sulfonierter Fettsäurealkylester, die bei hohen Feststoffgehalten auch bei mäßig erhöhten Temperaturen beweglich sind, durch Sulfonieren von Fettsäurealkylestern mit einem Sulfonierungsreagens und nachfolgende Aufarbeitung der Rohsulfonsäure in wäßrigem Medium unter Alkalimetallsalz-Bildung, das durch gekennzeichnet ist, daß man dem Reaktionsgemisch ein oder mehrere Hyroxyocenolsulfonate und/oder Hydroxyocenolethersulfonate zusetzt.

Das erfindungsgemäße Verfahren zur Herstellung niedrigviskoser, hochkonzentrierter wäßriger Pasten von Alkalimetallsalzen α-sulfonierter Fettsäurealkylester lehnt sich in mehreren wesentlichen Verfahrensschritten an entsprechende, aus dem Stand der Technik bekannte Verfahren zur Herstellung derartiger Verbindungen an. Als Ausgangsstoffe dienen im technischen Bereich heute Fette und/oder Öle natürlichen Ursprungs, die durch Esterspaltung und eine sich daran anschließende Veresterung mit niederen Alkanolen, bevorzugt mit Methanol, oder alternativ durch Umesterung von Triglyceriden natürlichen Ursprungs mit den entsprechenden niederen Alkanolen in großen Mengen preiswert hergestellt werden können. In bevorzugten Ausführungsformen werden dazu Fette und/oder Öle pflanzlichen Ursprungs verwendet, die ein für die Herstellung derartiger Verbindungen erwünschtes Spektrum der Kettenlänge der Alkylreste aufweisen.

Üblicherweise liegt dieses Spektrum im Bereich zwischen l0 und 24 C-Atomen im Fettsäurealkylrest. Es ist möglich, derartige Fettsäurealkylester als Reinverbindungen einzusetzen; im technischen Bereich werden jedoch häufig die aus der technischen Fett- bzw. Ölgewinnung resultierenden Fettsäureestergemische als Ausgangsstoffe verwendet. Die Sulfonierung derartiger Fettsäureester oder Fettsäureestergemische wird in Gegenwart eines üblichen Sulfonierreagens durchgeführt. Bevorzugt werden dazu gasförmiges $SO_3$ oder Mischungen mit Inertgasen (Luft, Stickstoff) verwendet, die mehr oder weniger große Mengen $SO_3$ enthalten. Alternativ dazu ist es auch möglich, andere Sulfonierreagenzien zur Sulfonierung zu verwenden. Als Beispiel sei Chlorsulfonsäure angeführt. Das Sulfoniermittel wird üblicherweise im molaren Überschuß eingesetzt. Dies bedeutet, daß pro Mol Fettsäurealkylester l,2 bis 2 Mol des Sulfoniermittels, bevorzugt an gasförmigem $SO_3$, eingesetzt werden. Die Sulfonierreaktion wird, um eine möglichst große Produktausbeute zu erreichen, bis zu mehr oder weniger hohen Sulfoniergraden geführt. Sulfoniergrade im Bereich von 95 % sind angestrebt. Im Einzelfall der Sulfonierung bestimmter Fettsäurealkylester bzw. Fettsäurealkylestergemische hängt jedoch der erreichbare Sulfoniergrad von zahlreichen Einzelfaktoren ab. Begrenzend ist in ersten Linie die bei hohen Sulfoniergraden häufig beobachtete Dunkelfärbung der Rohsulfonate, die im späteren Verfahrensablauf einen zusätzlichen Bleichschritt erfordert. Es wird daher im Einzelfall ein Kompromiß zu finden sein, um ein Optimum an Sulfoniergrad und Hellfarbigkeit des Produktes sicherzustellen.

Ein weiterer, in Abhängigkeit von der Farbe der erhaltenen a-Sulfofettsäureester vorzusehender Verfahrensschritt ist die Bleichung der Rohsulfonate, die üblicherweise durch Versetzen der Rohproduktmischungen mit Wasserstoffperoxid und/oder wäßrigen Hypochloritlösungen bewirkt wird. Gegebenenfalls kann auch noch eine Nachbleiche vor oder während der Endlagerung der schon neutralisierten Pasten erforderlich sein. Auch dieses Verfahren ist dem Fachmann aus dem Stand der Technik bekannt und bedarf daher an dieser Stelle keiner weiteren Erläuterung.

Ein ebenfalls an sich bekannter Verfahrensschritt liegt in dem Eintragen der gegebenenfalls schon gebleichten Rohsulfonate in wäßrige Alkalimetallhydroxidlösungen unter Salzbildung. Auch dieser Verfahrensschritt ergibt sich im wesentlichen aus den aus dem Stand der Technik bekannten Druckschriften und wird in

bevorzugter Weise dadurch bewirkt, daß man die gegebenenfalls gebleichten Rohsulfonate in eine vorgelegte wäßrige Lösung eines Alkalimetallhydroxids, vorzugsweise in eine wäßrige Natriumhydroxidlösung, einträgt. Die Lösung muß - um Konzentrations-und Temperaturspitzen zu vermeiden - intensiv durchmischt und gekühlt werden. Entsprechende Vorrichtungen für das großtechnische Arbeiten sind dem Fachmann ebenfalls aus dem Stand der Technik bekannt und bedürfen daher keiner weiteren Erläuterung.

Erfindungsgemäß werden in dem Verfahren zur Herstellung der wäßrigen Estersulfonatsalzpasten dem Reaktionsgemisch ein oder mehrere Hydroxyocenolsulfonate und/oder Hydroxyocenolethersulfonate zugesetzt. Dabei ist es möglich, Einzelverbindungen aus der Gruppe der Hydroxyocenolsulfonate oder Einzelverbindungen aus der Gruppe der Hydroxyocenolethersulfonate zuzusetzen. Es können jedoch auch mehrere Verbindungen aus einer Gruppe miteinander oder auch mit einer oder mehreren Verbindungen aus der anderen Gruppe gemeinsam eingesetzt werden. Entsprechend dem Herstellungsweg dieser Verbindungen, auf den unten näher eingegangen wird, entstehen diese in technischen Verfahren üblicherweise in Mischungen, so daß auch der Einsatz von Mischungen derartiger Verbindungen im technischen Verfahren zur Herstellung von Estersulfonatsalzpasten niedriger Viskosität bevorzugt ist.

In einer bevorzugten Ausführungsform der Erfindung setzt man dem Reaktionsgemisch eine oder mehrere Verbindungen der allgemeinen Formel (I) zu

$$CH_3(CH_2)_a-\overset{X}{\overset{|}{C}}H-(CH_2)_b-\overset{Y}{\overset{|}{C}}H-(CH_2)_c-CH_2O-Z_x-H \qquad (I)$$

In dieser allgemeinen Formel (I) bedeuten X und Y voneinander verschiedene Substituenten, die für OH oder $SO_3M$ stehen, worin M Wasserstoff, ein Alkalimetall, ein Erdalkalimetall oder einen Ammoniumrest bedeutet. Derartige Alkalimetalle sind in bevorzugter Weise Lithium, Natrium oder Kalium, von denen Natrium naturgemäß besonders bevorzugt ist. Als Erdalkalimetalle kommen Magnesium, Calcium, Strontium oder Barium in Frage, wobei zu berücksichtigen ist, daß dann ein Alkalimetallion zwei über $SO_3$-Gruppen gebundene Moleküle der allgemeinen Formel (I) bindet. Die Ammoniumreste können entweder $NH_4$-Reste oder $NR_4$-Reste sein, worin R wahlweise für Wasserstoff oder einen Alkylrest oder Hydroxyalkylrest mit 1 bis 4 C-Atomen stehen kann. In bevorzugten Ausführungsformen des erfindungsgemäßen Verfahrens werden solche Verbindungen der allgemeinen Formel (I) eingesetzt, in der X bzw. Y für eine $SO_3M$-Gruppe stehen, worin M Natrium oder Ammonium ($NH_4$) bedeutet.

In der allgemeinen Formel (I) steht Z für Ethoxygruppen, Propoxygruppen oder Butoxygruppen. Von derartigen Gruppen sind die Ethoxyreste bevorzugt.

x in der allgemeinen Formel (I) kann entweder für 0 stehen, wobei die Verbindungen der allgemeinen Formel (I), in denen x für 0 steht, Hydroxyocenolsulfonate sind, oder für Zahlen im Bereich von 1 bis 30 stehen, wobei derartige Verbindungen dann Hydroxyocenolethersulfonate sind. In dem erfindungsgemäßen Verfahren werden mit Vorzug solche Verbindungen der allgemeinen Formel (I) zugesetzt, in der x für Zahlen im Bereich von 0 bis 10 steht. Dies bedeutet, daß die bevorzugt zugesetzten Verbindungen entweder Hydroxyocenolsulfonate oder Hydroxyocenolethersulfonate sind, wobei letztere 1 bis 10 Ethoxygruppen am Molekülende enthalten.

In der oben angegebenen allgemeinen Formel (I) stehen außerdem a und c für Zahlen im Bereich von 0 bis 18 und b für 0, 1 oder 2, wobei die Summe (a + b + c) im Bereich der Zahlen von 12 bis 18 liegt. Bevorzugt werden solche Verbindungen der allgemeinen Formel (I) zugesetzt, in denen die Summe (a + b + c) 16 oder 18 ist. Die entsprechenden Hydroxyocenolsulfonate bzw. Hydroxyocenolethersulfonate leiten sich also von ungesättigten Fettsäuren wie beispielsweise Ölsäure und Linolsäure ab, die 16 bis 18 C-Atome im Alkylrest tragen.

Ein oder mehrer Hydroxyocenolsulfonate und/oder Hydroxyocenolethersulfonate werden in dem erfindungsgemäßen Verfahren zur Herstellung von α-Sulfofettsäurealkylestersalzen mit niedriger Viskosität dem Reaktionsgemisch zugesetzt. Dabei ist es möglich, ein oder mehrere Hydroxyocenolsulfonate allein oder in Mischungen mit einem oder mehreren Hydroxyocenolethersulfonaten der Rohsulfonsäure zuzusetzten, bevor diese in die wäßrige basische Lösung eingetragen wird. Entsprechend einer anderen Ausführungsform des Verfahrens ist es möglich, das bzw. die Hydroxyocenolsulfonat(e) und/oder Hydroxyocenolethersulfonat(e) zuzusetzen, während die Rohsulfonsäure in die wäßrige basische Lösung eingetragen wird. Eine weitere Ausführungsform des erfindungsgemäßen Verfahrens betrifft den Zusatz eines oder mehrerer Hydroxyocenolsulfonat(e) und/oder Hydroxyocenolethersulfonat(e) in die schon vereinigte Mischung von Rohsulfonsäure und wäßriger basischer Lösung, d.h. nach Einleiten der Rohsulfonsäure in die wäßrige basische Lösung. Bevorzugt ist ein Zusatz eines oder mehrerer Hydroxyocenolsulfonat(e) allein oder in Mischungen mit einem oder mehreren Hydroxyocenolethersulfonat(en) während der Einleitung der Rohsulfonsäure in das wäßrige basische Medium.

Durch Zusatz der oben näher beschriebenen Viskositätsregler aus der Gruppe der Hydroxyocenolsulfonate bzw. Hydroxyocenolethersulfonate wird in vorteilhafter Weise die Viskosität von hochkonzentrierten Estersulfonatsalzpasten erniedrigt. Dabei überrascht nicht nur die Tatsache, daß schon kleine Mengen zugesetzter Viskositätsregler für eine Erniedrigung der Viskosität verantwortlich sind, sondern auch die Tatasche, daß diese Mengen eine Absenkung der Viskosität auf Werte im Bereich von l0 bis 90 % des Viskositätswerts ermöglichen, den die Paste ohne Zusatz des Viskositätsreglers haben würde. So ist es in bevorzugten Ausführungsformen des erfindungsgemäßen Verfahrens möglich, die Viskosität drastisch zu senken, wenn man dem Reaktionsgemisch einen oder mehrere Hydroxyocenolsulfonate und/oder Hydroxyocenolethersulfonate in Gesamtmengen von l bis l0 Mol-% zusetzt. Mit Vorteil liegt die Gesamtmenge der genannten Viskositätsregler im Bereich von 2 bis 8 Mol-%. Derartige Zusätze ermöglichen eine Erniedrigung der Viskosität sowohl hochkonzentrierter, beispielsweise 60 %iger, Estersulfonatsalzpasten als auch niedrigerprozentiger, beispielsweise 30 %iger, Estersulfonatsalzpasten auf Werte, die deutlich unter 50 % des Viskositätswerts liegen, den die Paste ohne Zusatz der Viskositätsregler haben würde.

Die Herstellung der in dem erfindungsgemäßen Verfahren als Viskositätsregler eingesetzten Hydroxyocenolsulfonate und Hydroxyocenolethersulfonate erfolgt in aus dem Stand der Technik bekannter Weise. So wird beispielsweise in der deutschen Patentanmeldung P 37 25 030.2 ein Verfahren zur Herstellung der Hydroxyocenolsulfonate und Hydroxyocenolethersulfonate beschrieben. Darin werden Fettalkohole mit ungesättigten Alkylgruppen mit 9 bis l7 C-Atomen mit Olefinepoxiden, bevorzugt Ethylenoxid, Propylenoxid und/oder Butylenoxid, alkoxyliert und die erhaltenen Produkte mit einer Carbonsäure mit l bis 4 C-Atomen im Alkylrest verestert. Die erhaltenen ungesättigten Fettalkyl- oder Fettalkylpolyoxyalkylester, die überwiegend aus Fetten und/oder Ölen natürlicher Herkunft hergestellt werden und als Fettalkylrest überwiegend Oleylgruppen, Palmitoleylgruppen, Linoleylgruppen, Gadoleylgruppen und/oder Erucylgruppen enthalten und gegebenenfalls eine oder mehrere Alkoxygruppen am Kettenende aufweisen, werden dann mit einem Sulfonierungsreagenz, in erster Linie Schwefeltrioxid, umgesetzt. Das Umsetzungsprodukt wird in die wäßrige Lösung von l bis 2,5 Mol Alkalimetall-, Erdalkalimetall- oder Ammoniumhydroxid pro Mol angelagertes $SO_3$ eingetragen und diese Lösung bis zur Hydrolyse der enthaltenen Ester- und Sultongruppen erwärmt. In dem erfindungsgemäßen Verfahren werden derartige wäßrige Lösungen in möglichst konzentrierter Form eingesetzt. Die Hydroxyocenolsulfonate bzw. Hydroxyocenolethersulfonate mit Natrium- oder Ammoniumgruppen an der Sulfonatgruppe werden bevorzugt verwendet. Einsatzmengen der Viskositätsregler von l bis l0 Mol-%, besonders bevorzugt 2 bis 8 Mol-%, bewirken eine deutliche Erniedrigung der Viskosität von Estersulfonatsalzpasten.

Die Erfindung wird durch die nachfolgenden Beispiele näher erläutert.

In den nachfolgenden Beispielen wird die Viskosität der Estersulfonatsalzpasten in einem Epprecht-TV Viskosimeter bzw. in einem Brookfield-Viskosimeter gemessen. Die Erniedrigung der Viskosität durch das erfindungsgemäße Zusetzen von Viskositätsreglern wird nicht in absoluten Zahlen angegeben, da die in den beiden Viskosimetern bestimmten Viskositäten (mPa . s) von der gewählten Schergeschwindigkeit abhängig sind. Vielmehr wurde der in dem jeweiligen Viskosimeter bestimmte Viskositätswert der Estersulfonatsalzpaste ohne Viskositätsregler als "l00 % Viskosität" gesetzt und die relative Erniedrigung der Viskosität nach Zusatz der Viskositätsregler bestimmt. Die Messungen wurden zweimal wiederholt und die Ergebnisse aus allen drei Messungen gemittelt.

Beispiel l

70 g (0,08 Mol) einer disalz- und US-freien 33 %igen $C_{l6/l8}$ a-Estersulfonatpaste wurden mit l8,4 g (0,008 Mol, entsprechend einem l0 %igen Zusatz) einer 36 %igen Hydroxyocenol-l0EO-sulfonat-Na-Salz-Paste (I; X = OH; Y = $SO_3M$ mit M = Na; (a + b + c) = l4; Z = $-CH_2-CH_2O-$; x = ca. l0; WAS = 0,277 mval/l00 g; US = 8 %; $SO_4^{2-}$ = 2 %; $OAc^-$ = 3 %) versetzt und auf dem Dampfbad unter intensivem Rühren homogenisiert.

Um störende Luftblasen zu entfernen, wurde das Homogenisat 30 min zentrifugiert, anschließend im Dampfbad auf die Meßtemperatur von 45 ^C gebracht und die Viskosität in einem Epprecht-TV-Viskosimeter (Meßkörper 2-4) bestimmt.

Ermittelte Viskositäten:

a) a-Estersulfonat (a-ES; Texin ES 68 der Fa. Henkel), 33 %ig
7 620 mPa.s = 100 %

b) a-ES + 10 Gew.-% HOES10-Na-Salz, 36 %ig
800 mPa.s = 11 %

Beispiel 2:

70 g (0,l4 Mol) einer 56 %igen disalz- und US-freien a-Estersulfonatpaste wurden mit 8,9 g (0,007 Mol, entsprechend einem 5 Gew.-%igen Zusatz) einer 65 %igen Hydroxyocenol-l0EO-sulfonat-Na-Salz-Paste (I; X = OH; Y = $SO_3M$ mit M = Na; (a + b + c) = l4; Z = -$CH_2$-$CH_2O$-; x = ca. l0; WAS = 0,53 mval/l00g; US = l2 %; $SO_4^{2-}$ = 5 %; $OAc^-$ = 4 %) versetzt und auf dem Dampfbad unter intensivem Rühren homogenisiert.

Um störende Luftblasen zu entfernen, wurde das Homogenisat 30 min zentrifugiert, anschließend im Dampfbad auf die Meßtemperatur von 90 ^C gebracht und die Viskosität in einem Brookfield-Viskosimeter (Spindel l, 60 U/min) bestimmt.

Ermittelte Viskositäten:

a) a-Estersulfonat (a-ES; Texin ES 68 der Fa. Henkel), 56 %ig
5 800 mPa.s = 100 %

b) a-ES + 5 Gew.-% HOES10-Na-Salz, 65 %ig
2 400 mPa.s = 41 %

Vergleichsbeispiel l:

In einem Blindversuch wurden 70 g (0,08 Mol) einer disalz- und US-freien 33 %igen a-Estersulfonatpaste l0 Gew.-% Wasser zugesetzt und die Viskosität entsprechend Beispiel l bestimmt.

Ermittelte Viskositäten:

| | |
|---|---|
| a-Estersulfonat (Texin ES 68), 33 %ig | 7 620 mPa.s = 100 % |
| a-ES + 10 Gew.-% $H_2O$ | 6 860 mPa.s = 90 % |

Vergleichsbeispiel 2:

In der in Vergleichsbeispiel l beschriebenen Weise wurde der 33 %igen α-Estersulfonatpaste 5 Gew.-% Wasser zugesetzt und die Viskosität entsprechend Beispiel l bestimmt. Die ermittelten relativen Viskositätswerte sind der nachfolgenden Tabelle zu entnehmen.

Beispiel 3:

Der in Beispiel l beschriebenen α-Estersulfonatpaste (33 % AS) wurde in Mengen von 5 und l0 Gew.-% ein Hydroxyocenolsulfat der allgemeinen Formel (I) (mit X = OH; Y = $SO_3M$ mit M = Na; (a + b + c) = l4) bzw. 5 Gew.-% eines Hydroxyocenolethersulfonats der allgemeinen Formel (I) (mit X = OH; Y = $SO_3M$ mit M = Na; Z = -$CH_2$-$CH_2O$-; (a + b + c) = l4: x = ca. l0) zugesetzt und die Viskosität in der Weise bestimmt, wie dies in

Beispiel I beschrieben wurde. Die Ergebnisse sind der nachfolgenden Tabelle zu entnehmen.

Beispiel 4:

In der in Beispiel 2 beschriebenen Weise wurde die dort angegebene 56 %ige α-Estersulfonatpaste mit 5 bzw. l0 Gew.-% des Hydroxyocenolsulfonats (I) bzw. mit l0 Gew.-% des Hydroxyocenolethersulfonats (I) versetzt, die in Beispiel 3 definiert wurden. Die relative Viskosität wurde in der in Beispiel 2 beschriebenen Weise bestimmt. Die Ergebnisse sind der nachfolgenden Tabelle zu entnehmen.

Vergleichsbeispiele 3 und 4:

In einem weiteren Blindversuch wurden 70 g (0,l4 Mol) einer 56 %igen disalz- und US-freien α-Estersulfonatpaste mit 5 bzw. l0 Gew.-% Wasser versetzt und die Viskosität in der in Beispiel 2 beschriebenen Weise bestimmt. Die relativen Viskositäten sind ebenfalls der nachfolgenden Tabelle zu entnehmen.

Tabelle

| Bsp. | Texin ES68 AS (%) | Meth.[1] | HOS[2] Gew.-% | HOES[3] Gew.-% | $H_2O$ Gew.-% | Rel. Visk. % |
|---|---|---|---|---|---|---|
| 1a | 33 | A | - | - | - | 100 |
| 3a | 33 | A | 5 | - | - | 15 |
| 3b | 33 | A | 10 | - | - | 8 |
| 3c | 33 | A | - | 5 | - | 6 |
| 1b | 33 | A | - | 10 | - | 11 |
| Vgl.2 | 33 | A | - | - | 5 | 92 |
| Vgl.1 | 33 | A | - | - | 10 | 90 |
| 2a | 56 | B | - | - | - | 100 |
| 4a | 56 | B | 5 | - | - | 87 |
| 4b | 56 | B | 10 | - | - | 59 |
| 2b | 56 | B | - | 5 | - | 41 |
| 4c | 56 | B | - | 10 | - | 45 |
| Vgl.3 | 56 | B | - | - | 5 | 54 |
| Vgl. 4 | 56 | B | - | - | 10 | 55 |

Anmerkungen:

1) Methode A:
- Epprecht-TV-Viskosimeter
- 45 °C
- Meßkörper 2-4

Methode B:
- Brookfield-Viskosimeter
- 90 °C
- Spindel 1
- 60 U/min

2) HOS = Hydroxyocenolsulfonat I mit X = OH; Y = $SO_3M$ mit M = Na; (a + b + c)= 14

3) HOES10 = Hydroxyocenolethersulfonat I mit X = OH; Y = $SO_3M$ mit M = Na; Z = $-CH_2CH_2O-$; (a + b + c) = 14; x = ca. 10

Beispiel 5

In einem l Liter-Sulfonierreaktor mit Gaseinleitungsrohr und Mantelkühlung wurden 200 g (0,88 mol) Palmstearinsäuremethylester (IZ = 0,08, VZ = 198,4, SZ = 0,45, M = 228,2) vorgelegt und bei 80°C mit 84 g (1,05 mol) $SO_3$, entsprechend einem Verhältnis Ester : $SO_3$ = 1 : 1,2, umgesetzt.

Das $SO_3$ wurde durch Erhitzen aus einer entsprechenden Menge Oleum ausgetrieben, mit Stickstoff auf eine Konzentration von 5 Vol.% verdünnt und innerhalb von 26 min in den Methylester eingeleitet, wobei die Temperatur des Reaktionsgemisches durch intensive Kühlung stets unter 90°C gehalten wurde.

Nach der Sulfonierung wurde das saure Reaktionsprodukt zunächst 25 min bei 80°C getempert, anschließend auf l5°C abgekühlt, mit 40 g, entsprechend 8 Gew.-% einer 56%igen wässrigen Lösung von Hydroxyocenol-l0EO-sulfonat-Na-Salz versetzt und in eine Lösung von 44 g (l,l mol) NaOH in ca 500 ml Wasser eingerührt.

Im Anschluß wurden 70 g der auf diesem Wege gewonnenen Paste (AS-Gehalt = 40,4 %) durch intensives Rühren homogenisiert, zur Entfernung von Luftblasen 30 min zentrifugiert, auf dem Dampfbad auf die Meßtemperatur von 45°C gebracht und die Viskosität in einem Epprecht-TV-Viskosimeter (Meßkörper 3-4) bestimmt.

Der Vergleich erfolgte gegen eine Probe, der während der Neutralisation kein Tensid zur Viskositätssenkung zugesetzt wurde.

Ermittelte Viskositäten:

| | |
|---|---|
| a) alpha-Estersulfonat (alpha-ES, 40%ig) | 9480 mPa.s<br>100 % |
| b) alpha-ES + 8 Gew.-% HOES 10-Na-Salz, 36%ig | 1990 mPa.s<br>21% |

<u>Vergleichsbeispiel 5</u>

In einem Vergleichsversuch wurden 70 g der 40,4 %igen Paste mit 8 Gew.-% Wasser versetzt und die Viskositäten in der in Beispiel 5 beschriebenen Weise bestimmt:

Ermittelte Viskositäten:

| | |
|---|---|
| a) alpha-ES (40 %ig) | 9480 mPa.s<br>100 % |
| b) alpha-ES + 8 Gew.-% $H_2O$ | 6420 mPa.s<br>68 % |

## Patentansprüche

1. Verfahren zur Herstellung niedrigviskoser, hochkonzentrierter wäßriger Pasten von Alkalimetallsalzen alpha-sulfonierter Fettsäurealkylester, die bei hohen Feststoffgehalten auch bei mäßig erhöhten Temperaturen beweglich sind, durch Sulfonieren von Fettsäurealkylestern mit einem Sulfonierungsreagenz und nachfolgende Aufarbeitung der Rohsulfonsäure in wäßrigem Medium unter Alkalimetallsalz-Bildung, dadurch gekennzeichnet, daß man dem Reaktionsgemisch ein oder mehrere Hydroxyocenolsulfonat(e) und/oder Hydroxyocenolethersulfonat(e) zusetzt.

2. Verfahren nach Anspruch l, dadurch gekennzeichnet, daß man dem Reaktionsgemisch eine oder mehrere Verbindungen der allgemeinen Formel (I) zusetzt

$$CH_3(CH_2)_a-\overset{X}{\overset{|}{C}}H-(CH_2)_b-\overset{Y}{\overset{|}{C}}H-(CH_2)_c-CH_2O-Z_x-H \qquad (I)$$

in der

X und Y unterschiedlich sind und für OH oder $SO_3M$ stehen, worin M H, Alkalimetall, Erdalkalimetall oder Ammonium $NR_4$ (mit R = Wasserstoff oder Alkylrest oder Hydroxyalkylrest mit I bis 4 C-Atomen) bedeutet,

Z für Ethoxy-, Propoxy- oder Butoxygruppen steht und

a und c für Zahlen im Bereich von 0 bis I8,

b für 0, I oder 2 und

x für 0 (Hydroxyocenolsulfonate) oder Zahlen im Bereich von I bis 30 (Hydroxyocenolethersulfonate) stehen, wobei die Summe (a + b + c) im Bereich der Zahlen von I2 bis I8 liegt.

**3.** Verfahren nach Ansprüchen I und 2, dadurch gekennzeichnet, daß man dem Reaktionsgemisch eine oder mehrere Verbindungen der allgemeinen Formel (I) zusetzt, in der X und Y unterschiedlich sind und für OH oder $SO_3M$ stehen, worin M Natrium oder Ammonium ($NH_4$) bedeutet.

**4.** Verfahren nach Ansprüchen I und 2, dadurch gekennzeichnet, daß man dem Reaktionsgemisch ein oder mehrere Verbindungen der allgemeinen Formel (I) zusetzt, in der die Summe (a + b + c) I6 oder I8 ist.

**5.** Verfahren nach Ansprüchen I und 2, dadurch gekennzeichnet, daß man dem Reaktionsgemisch eine oder mehrere Verbindungen der allgemeinen Formel (I) zusetzt, in der x für Zahlen im Bereich von 0 bis I0 steht.

**6.** Verfahren nach Ansprüchen I und 2, dadurch gekennzeichnet, daß man dem Reaktionsgemisch eine oder mehrere Verbindungen der allgemeinen Formel (I) zusetzt, in der Z für Ethoxyreste steht.

**7.** Verfahren nach Ansprüchen I bis 6, dadurch gekenzeichnet, daß man dem Reaktionsgemisch eine oder mehrere Verbindungen der allgemeinen Formel (I) zusetzt,

$$CH_3(CH_2)_a-\overset{X}{\overset{|}{C}}H-(CH_2)_b-\overset{Y}{\overset{|}{C}}H-(CH_2)_c-CH_2O-Z_x-H \qquad (I)$$

in der

X und Y unterschiedlich sind und für OH oder $SO_3M$ stehen, worin M Natrium oder Ammonium ($NH_4$) bedeutet, und

die Summe (a + b + c) I6 oder I8 ist und

x für Zahlen im Bereich von 0 bis I0 und

Z für Ethoxyreste

stehen.

**8.** Verfahren nach Ansprüchen I bis 7, dadurch gekennzeichnet, daß man ein oder mehrere Hydroxyocenolsulfonat(e) und/oder ein oder mehrere Hydroxyocenolethersulfonat(e) vor, während oder nach der Einleitung der Rohsulfonsäure in das wäßrige Medium zusetzt.

**9.** Verfahren nach Anspruch 8, dadurch gekennzeichnet, daß man ein oder mehrere Hydroxyocenolsulfonat(e) und/oder ein oder mehrere Hydroxyocenolethersulfonat(e) während der Einleitung der Rohsulfonsäure in das wäßrige Medium zusetzt.

**10.** Verfahren nach Ansprüchen I bis 9, dadurch gekennzeichnet, daß man ein oder mehrere Hydroxyocenolsulfonat(e) und/oder Hydroxyocenolethersulfonat(e) in Gesamtmengen von I bis 10 Mol-%, bevorzugt von 2 bis 9 Mol-%, zusetzt.

## Claims

**1.** A process for the production of low-viscosity, highly concentrated water-containing pastes of alkali metal salts of a-sulfonated fatty acid alkyl esters which, despite high solids contents, are mobile even at moderately elevated temperatures by sulfonation of fatty acid alkyl esters with a sulfonating agent and subsequent working up of the crude sulfonic acid in aqueous medium with formation of an alkali metal salt,

characterized in that one or more hydroxyocenol sulfonate(s) and/or hydroxyocenol ether sulfonate(s) is/are added to the reaction mixture.

2. A process as claimed in claim 1, characterized in that one or more compounds corresponding to general formula (I)

$$CH_3(CH_2)_a-\overset{\overset{X}{|}}{C}H-(CH_2)_b-\overset{\overset{Y}{|}}{C}H-(CH_2)_c-CH_2O-Z_x-H \qquad (I)$$

in which

X and Y are different and represent OH or $SO_3M$ where M is H, alkali metal, alkaline earth metal or ammonium $NR_4$ (with R = hydrogen or a $C_{1-4}$ alkyl or hydroxyalkyl radical ),

Z represents ethoxy, propoxy or butoxy groups and

a and c are numbers of 0 to 18,

b is the number 0, 1 or 2 and

x is the number 0 (hydroxyocenol sulfonates) or a number of 1 to 30 (hydroxyocenol ether sulfonates), the sum (a + b + c) being a number of 12 to 18.

3. A process as claimed in claims 1 and 2, characterized in that one or more compounds corresponding to general formula (I), in which X and Y are different and represent OH or $SO_3M$ where M is sodium or ammonium ($NH_4$), is/are added to the reaction mixture.

4. A process as claimed in claims 1 and 2, characterized in that one or more compounds corresponding to general formula (I), in which the sum (a + b + c) is 16 or 18, is/are added to the reaction mixture.

5. A process as claimed in claims 1 and 2, characterized in that one or more compounds corresponding to general formula (I), in which x is a number of 0 to 10, is/are added to the reaction mixture.

6. A process as claimed in claims 1 and 2, characterized in that one or more compounds corresponding to general formula (I), in which Z represents ethoxy groups, is/are added to the react ion mixture.

7. A process as claimed in claims 1 to 6, characterized in that one or more compounds corresponding to general formula (I)

$$CH_3(CH_2)_a-\overset{\overset{X}{|}}{C}H-(CH_2)_b-\overset{\overset{Y}{|}}{C}H-(CH_2)_c-CH_2O-Z_x-H \qquad (I)$$

in which

X and Y are different and represent OH or $SO_3M$ where M is sodium or ammonium ($NH_4$),

the sum (a + b + c) is 16 or 18,

x is a number of 0 to 10 and

Z represents ethoxy groups,

is/are added to the reaction mixture.

8. A process as claimed in claims 1 to 7, characterized in that one or more hydroxyocenol sulfonate(s) and/or one or more hydroxyocenol ether sulfonates is/are added before, during or after introduction of the crude sulfonic acid into the aqueous medium.

9. A process as claimed in claim 8, characterized in that one or more hydroxyocenol sulfonate(s) and/or one or more hydroxyocenol ether sulfonate(s) is/are added during introduction of the crude sulfonic acid into the aqueous medium.

**10.** A process as claimed in claims 1 to 9, characterized in that one or more hydroxyocenol sulfonate(s) and/or hydroxyocenol ether sulfonate(s) is/are added in total quantities of 1 to 10% mol-% and preferably 2 to 8 mol-%.

## Revendications

1) Procédé d'obtention de pâtes aqueuses à forte concentration et à viscosité réduite, d'esters d'alcoyle d'acide gras a-sulfonés qui sont mobiles pour des teneurs élevées en solides également à des températures modérément élevées, par sulfonation d'esters d'alcoyle d'acides gras avec un réactif de sulfonation et transformation subséquente de l'acide sulfonique brut en milieu aqueux avec formation de sel de métal alcalin, caractérisé en ce que l'on ajoute au mélange réactionnel un ou plusieurs hydroxyocénolsulfonates et/ou hydroxyocénoléthersulfonates.

2) Procédé selon la revendication 1, caractérisé en ce que l'on ajoute au mélange réactionnel un ou plusieurs composés de formule générale (I)

$$CH_3\ (CH_2)_a-\overset{\overset{X}{|}}{C}H-(CH_2)_b-\overset{\overset{Y}{|}}{C}H-(CH_2)_c-CH_2O-Z_x-\ H \qquad (I)$$

dans laquelle

X et Y sont différents et représentent OH ou $SO_3M$, dans lequel M signifie un métal alcalin, un métal alcalino-terreux ou un ammonium $NR_4$ (avec R = hydrogène ou un radical alcoyle ou un radical hydroxyalcoyle ayant de 1 à 4 atomes de carbone,

Z représente un groupe éthoxy, un groupe propoxy ou un groupe butoxy et

a et c représentent des nombres entiers dans la plage de 0 à 18,

b représente 0, 1 ou 2 et

x représente zéro (hydroxyocénolsulfonates) ou des nombres dans la plage de 1 à 30 (hydroxyocénoléthersulfonates) pour lesquels la somme (a + b + c) se situe dans la plage des nombres allant de 12 à 18.

3) Procédé selon les revendications 1 et 2, caractérisé en ce que l'on ajoute au mélange réactionnel un ou plusieurs composés de formule générale (I) dans laquelle X et Y sont différents et représentent OH ou $SO_3M$, dans lequel M signifie du sodium ou de l'ammonium ($NH_4$).

4) Procédé selon les revendications 1 et 2, caractérisé en ce que l'on ajoute au mélange réactionnel un ou plusieurs composés de formule générale (I) dans laquelle la somme (a + b + c) est 16 ou 18.

5) Procédé selon les revendications 1 et 2, caractérisé en ce que l'on ajoute au mélange réactionnel un ou plusieurs composés de formule générale (I) : dans laquelle x représente des nombres dans la plage de 0 à 10.

6) Procédé selon les revendications 1 et 2, caractérisé en ce que l'on ajoute au mélange réactionnel un ou plusieurs composés de formule générale (I) dans laquelle Z représente un radical éthoxy.

7) Procédé selon les revendication 1 à 6, caractérisé en ce que l'on ajoute au mélange réactionnel un ou plusieurs composés de formule générale (I)

$$CH_3\ (CH_2)_a-\overset{\overset{X}{|}}{C}H-(CH_2)_b-\overset{\overset{Y}{|}}{C}H-(CH_2)_c-CH_2O-Z_x-\ H \qquad (I)$$

dans laquelle X et Y sont différents et représentent OH ou $So_3M$ dans lequel M signifie du sodium ou de l'ammonium, et la somme (a + b + c) est 16 ou 18, et X représente des nombres dans la plage allant de 0 à 10 et Z représente un radical éthoxy.

8) Procédé selon les revendications 1 à 7, caractérisé en ce que l'on ajoute un ou plusieurs hydroxyocénolsulfonates et/ou un ou plusieurs hydroxyocénoléthersulfonates avant, pendant, ou après l'introduction de l'acide sulfonique brut dans le milieu aqueux.

9) Procédé selon la revendication 8, caractérisé en ce que l'on ajoute un ou plusieurs hydroxyocénolsulfonates et/ou un ou plusieurs hydroxyocénoléthersulfonates pendant l'introduction de l'acide sulfonique brut

dans le milieu aqueux.

10) Procédé selon les revendications 1 à 9, caractérisé en ce que l'on ajoute un ou plusieurs hydroxyocénolsulfonates et/ou hydroxyocénoléthersulfonates en quantités totales allant de 1 à 10 % molaire, de préférence de 2 à 8 % molaire.